Europäisches Patentamt

European Patent Office

Office européen des brevets

(19)

(11) Veröffentlichungsnummer: **0 059 868**
**B1**

(12) **EUROPÄISCHE PATENTSCHRIFT**

(45) Veröffentlichungstag der Patentschrift:
**02.05.85**

(21) Anmeldenummer: **82101259.8**

(22) Anmeldetag: **19.02.82**

(51) Int. Cl.⁴: **A 61 N 1/36,** A 61 B 5/14,
G 05 B 13/02

(54) **Einrichtung zur Regelung der Stimulationsfrequenz von Herzschrittmachern.**

(30) Priorität: **26.02.81 DE 3107128**

(43) Veröffentlichungstag der Anmeldung:
**15.09.82 Patentblatt 82/37**

(45) Bekanntmachung des Hinweises auf die Patenterteilung:
**02.05.85 Patentblatt 85/18**

(84) Benannte Vertragsstaaten:
**CH DE FR GB IT LI NL SE**

(56) Entgegenhaltungen:
**DE - A - 2 609 365**
**US - A - 4 009 721**

**IEEE TRANS. ON BIOMEDICAL ENGINEERING; Band BME-24, Nr. 2, Marz 1977, Seiten 195-197, New York, USA**

(73) Patentinhaber: **Wirtzfeld, Alexander, Hauptstrasse 26b, D-8191 Thanning (DE)**
Patentinhaber: **Heinze, Roland, Wilhelm Diess Weg 13, D-8000 München 81 (DE)**
Patentinhaber: **Bock, Thomas, Kafkastrasse 48, D-8000 München 83 (DE)**
Patentinhaber: **Liess, Hans Dieter, Fasanenstrasse 106, D-8025 Unterhaching (DE)**

(72) Erfinder: **Wirtzfeld, Alexander, Hauptstrasse 26b, D-8191 Thanning (DE)**
Erfinder: **Heinze, Roland, Wilhelm Diess Weg 13, D-8000 München 81 (DE)**
Erfinder: **Bock, Thomas, Kafkastrasse 48, D-8000 München 83 (DE)**
Erfinder: **Liess, Hans Dieter, Fasanenstrasse 106, D-8025 Unterhaching (DE)**

(74) Vertreter: **Hansmann, Dierk, Dipl.-Ing., Jessenstrasse 4, D-2000 Hamburg 50 (DE)**

## Beschreibung

Die Erfindung betrifft eine Einrichtung zur Frequenzregelung eines Herzschrittmachers in Abhängigkeit von der gemessenen Änderung der zentralvenösen Blut-Sauerstoffsättigung. Sie findet somit Anwendung bei allen Patienten, die einen Herzschrittmacher benötigen.

Die Erfindung dient dem Ziel, bei Herzschrittmacherpatienten den Sauerstoffbedarf des Körpers über den Blutkreislauf dadurch optimal zu decken, dass sich die Schlagfrequenz des Herzens wie im natürlichen Fall den jeweiligen Belastungsverhältnissen anpasst, wobei die optimale hämodynamische Situation vom Schrittmacher selbst gefunden wird.

Sie hat zugleich den Zweck, trotz der erweiterten Schrittmacherfunktion, die bisherige bewährte Ausführungsform der Herzschrittmacher und der zugehörigen Schrittmacherkatheder praktisch nicht zu verändern, so dass sowohl die bekannten Implantationstechniken gleichbleiben, als auch die hohen Anforderungen bezüglich des Langzeiteinsatzes erfüllt werden können.

Es ist bereits nach der DE-OS 27 17 659 bekannt, beim Herzschrittmacher die Schrittmacherfrequenz über eine Messung der zentralvenösen Sauerstoffsättigung zu steuern und so den jeweiligen Belastungsverhältnissen anzupassen. Dabei wird die Messung der Blutsauerstoffsättigung mit Hilfe einer Lichtleitersonde durchgeführt, die in den Stimulationskatheder eingebaut ist. Das dabei verwendete Messprinzip der Reflexionsoximetrie beruht auf der Bestimmung der Reflexionsintensitäten von Licht der Messwellenlänge von 660 nm (R660) und der Referenzwellenlänge von ca. 800 nm (R800) im Blut.

Kennzeichen der angewandten Frequenzanpassung ist eine feste Zuordnung der Schrittmacherfrequenz f zum jeweils ermittelten Messwert der Sauerstoffsättigung $S_{02}$

$$f = k \cdot S_{02}, \text{ wobei } f_{min} < f < f_{max}.$$

Die Werte für k, $f_{min}$ und $f_{max}$ müssen vor der Implantation fest eingestellt werden.

Weiterhin ist aus der DE-OS 21 13 247 bekannt, dass das Verhältnis der beiden Messwerte $R_{800}/R_{660}$ direkt proportional zur Blutsauerstoffsättigung $S_{02}$ ausgedrückt durch das Verhältnis $\dfrac{Hb02}{Hb02 + Hb}$ ist (wobei Hb für Hämoglobin und Hb02 für Oxyhämaglobin steht).

Die nach dem in der DE-OS 27 17 659 angegebenen Prinzip ausgeführten Herzschrittmacher zeigen eine Reihe von Problemen, die einem klinischen Einsatz derartiger Schrittmacher bisher im Wege stehen. Die Messmethode verträgt keine Veränderung auf der optischen Übertragungsstrecke (Lichtleiter, Reflexionsraum und Koppelstellen,) die eine wellenlängenabhängige Signalbeeinflussung verursachen. Dazu kann es kommen durch:

– schadhafte Koppelstellen

– Materialveränderungen am Lichtleiter
– Ablagerungen auf der Lichtöffnung am Katheder
– Fremdobjekte im Reflexionsbereich (Herzwand, Trabekeln)

Weiterhin kann sich die Steuerung der Schrittmacherfrequenz in Abhängigkeit von der Sauerstoffsättigung nach einer fest vorgegebenen Kennlinie nachteilig auswirken, wenn bei Fortschreiten der kardialen Grunderkrankung eine Änderung der Beziehung Herzleistung zu Herzfrequenz eintritt. Durch zu starkes Anheben der Frequenz kann es sogar zu einer Verschlechterung der Hämodynamik kommen.

Das Mess- und Steuerprinzip erfordert eine Eichung vor der Implantation und erhöht dementsprechend die Bedienanforderungen.

Ausserdem besitzt der Katheder nur eine begrenzte Funktionstüchtigkeit, da die Endhalterungen der Lichtleiter langfristig instabil werden, weil sie den grössten Teil der auf den Katheder wirkenden Zugbelastungen aufnehmen müssen.

Zudem ist die Wechselfestigkeit der Lichtleiter für den Langzeiteinsatz mit den heutigen Materialien nicht gegeben, und die Notwendigkeit, beim Kombikatheder die Lichtöffnung seitlich anzubringen, lässt technisch keinen Spielraum zur Weiterführung eines Mandarins (Stahldraht, der bei der Implantation in den hochelastischen Katheder geschoben wird, um die Einführung des Katheders in die Herzkammer zu erleichtern) über diese Stelle hinaus. Darüber hinaus ist das Koppelsystem zwischen Kombikatheder und Schrittmacher um ein vielfaches aufwendiger in der Herstellung, empfindlicher im Einsatz und voluminöser als bei konventioneller Schrittmachertechnik.

Da die Energieverluste bei dem Einsatz der Lichtleitertechnik und dem erwähnten Messprinzip nur mit hoher optischer Präzision klein zu halten sind, steigen auch die Kosten für das Gesamtsystem gegenüber konventioneller Technik auf ein mehrfaches.

Der Erfindung liegt die Aufgabe zugrunde, die beschriebenen Hindernisse zu überwinden und sowohl die Messung zur Bestimmung des Blutsauerstoffs als auch die Regelung der Stimulationsfrequenz apparativ so zu gestalten, dass eine langfristige, ungestörte Messwerterfassung und die bestmögliche hämodynamische Situation im Blutkreislauf garantiert ist und zugleich die Betriebssicherheit nicht verringert, sondern möglichst erhöht wird, wobei technisch langfristig bewährte, unkritische Bedien- und Herstellungspraktiken zum Einsatz kommen sollen.

Die Lösung dieder Aufgabe erfolgt durch die Merkmale des Anspruches 1. Weitere Ausgestaltungen der Erfindung sind durch die Merkmale der Ansprüche 2 bis 17 gekennzeichnet.

Die Vorteile, die sich hierdurch erzielen lassen, bestehen darin:

– Mit der erfindungsgemässen Messonde M ist der Katheder in seinem mechanischen Aufbau praktisch identisch mit den seit langem im Einsatz befindlichen bipolaren Kathedern und bringt dementsprechend keine zusätzlichen Probleme bezüglich der mechanischen Langzeitfestigkeit und der Implantationstechnik und besitzt die besten Eigenschaften bezüglich einer langfristigen optischen Erfassung der Blutsauerstoffsättigung.

- Die Messwerterfassung ermöglicht es, mit nur zwei elektrischen Leitungen (Drahtwedeln) im Katheder auszukommen und damit die bewährten Kathedertechniken zu nutzen.

- Die Messwertverarbeitung ermöglicht es, unabhängig von sehr kurzen oder langfristigen Veränderungen auf der Messstrecke normale zeitliche Schwankungen der körperlichen Belastung des Patienten mit gleichbleibender Genauigkeit zu erfassen.

- Das Regelsystem zur Anpassung der Stimulationsfrequenz an die Belastungen des Patienten ermöglicht sowohl eine direkte Nachregelung bei Belastungsschwankungen als auch eine autonome Optimalregelung im Sinne einer bestmöglichen Sauerstoffversorgung bei möglichst geringer Herzbelastung, also möglichst niedriger Stimulationsfrequenz.

- Die Fehlerdetektion ermöglicht die Erkennung von Störungsfällen bei der Messwerterfassung und -Auswertung sowie bei Bruch der elektrischen Leitungen im Katheder und die Nutzung von zwei Leitungen zur lebenswichtigen Stimulation – und erhöht damit die Funktionssicherheit.

Ein Ausführungsbeispiel der Erfindung ist in den Zeichnungen dargestellt und wird in folgendem näher beschrieben. Es zeigen:

Figur 1 eine Anordnung eines Herzschrittmachers mit Stimulationskatheder und Messonde zur Reizung des Herzmuskels,

Figur 2 einen Längsschnitt durch eine Messonde mit konzentrisch angeordneten Drahtwedeln,

Figur 3 einen Längsschnitt durch eine weitere Ausführungsform einer Messonde mit parallel angeordneten Drahtwedeln,

Figur 4 einen Schnitt gemäss Linie IV–IV der Figur 3,

Figur 5 einen Schnitt gemäss Linie V–V der Figur 3,

Figur 6 eine Ansteuerschaltung der Messonde,

Figur 7 eine Schaltung der Messonde,

Figur 8 ein Diagramm der Strom-Spannungskennlinien der Messonde,

Figur 9 ein Blockschaltbild für eine analoge Signal-Wandler-Schaltung,

Figur 10 ein Blockschaltbild eines Herzschrittmachers,

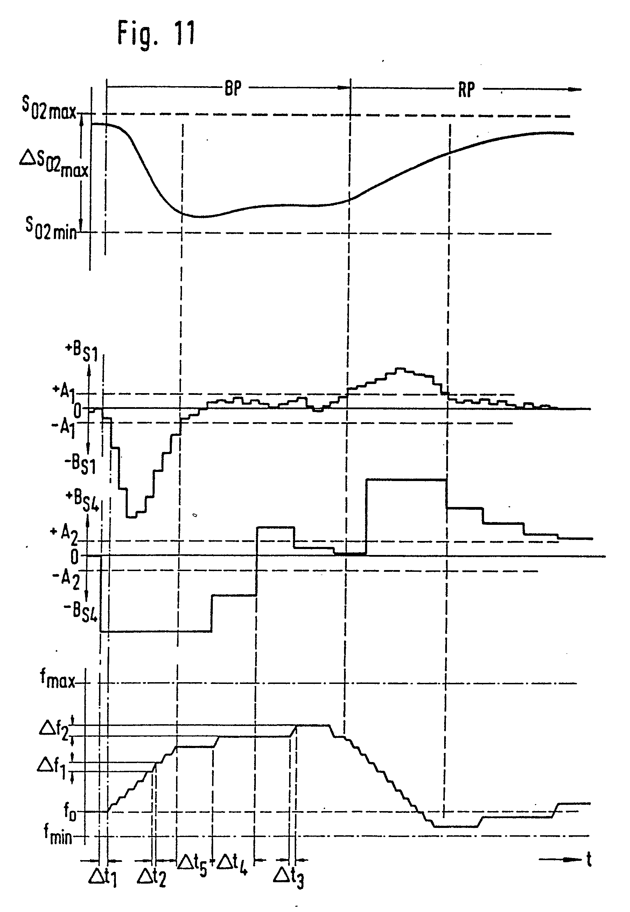

Figur 11 ein Diagramm des zeitlichen Verlaufs der Stimulationsfrequenz in Abhängigkeit von der zentralvenösen Blutsauerstoffsättigung.

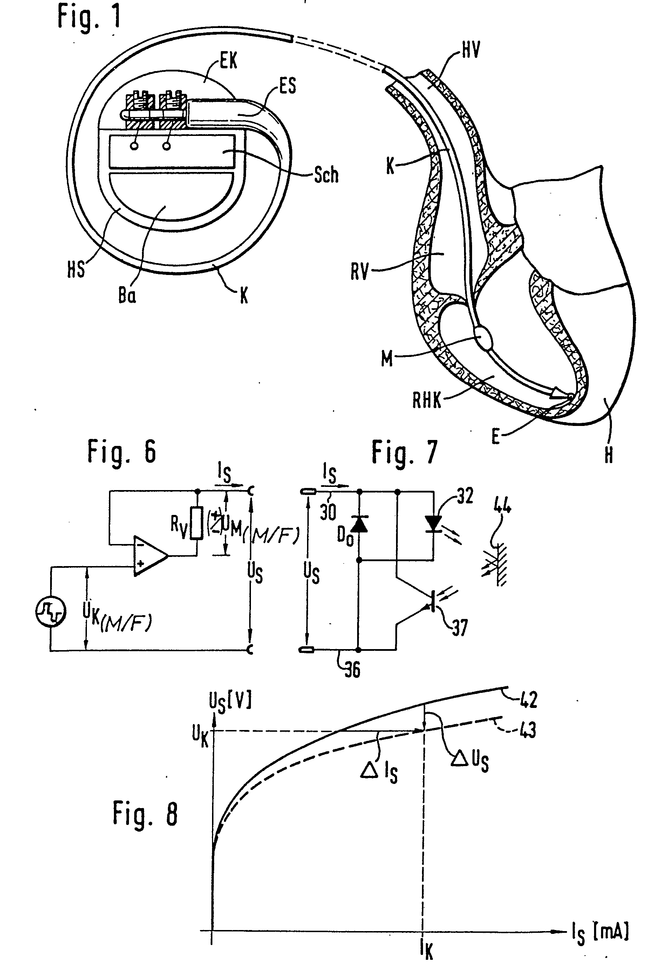

Gemäss Figur 1 enthält der Herzschrittmacher HS den Stromversorgungsteil Ba, den elektronischen Schaltungsteil Sch und die zweipolige elektrische Kupplung EK. In der Kupplung EK ist der zweipolige Stecker ES des Stimulationskatheders K festgeschraubt. Der Katheder führt über die obere Hohlvene HV in den rechten Vorhof RV und in die rechte Herzkammer RHK, so dass dort von der Messonde M die Blutsauerstoffsättigung gemessen und durch die Stimulationselektrode E der Herzmuskel H gereizt wird.

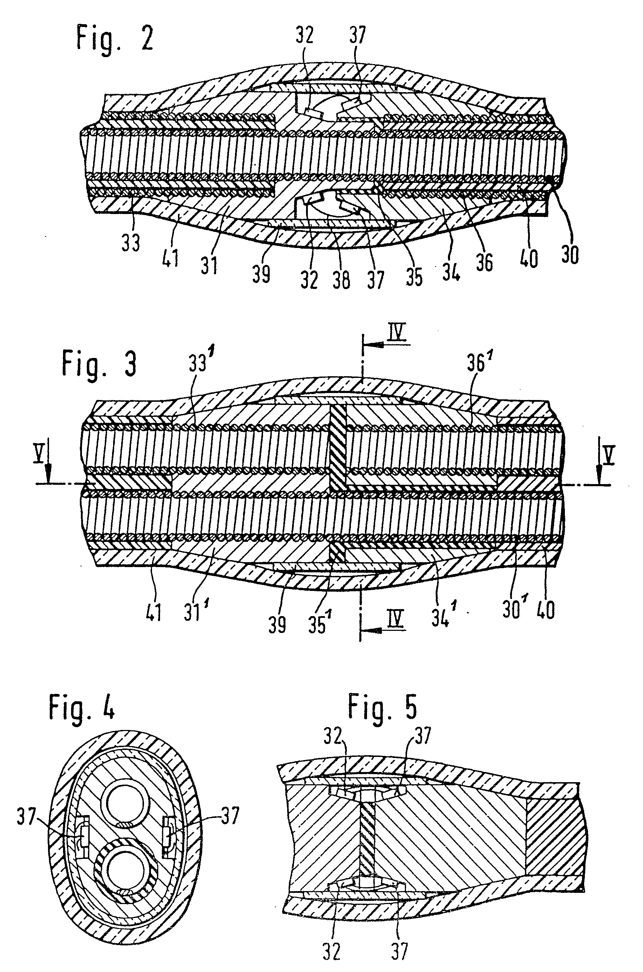

Gemäss Figur 2 bis 5 sind zwei Ausführungsformen näher gezeigt. Von den heute im Einsatz befindlichen bipolaren Stimulationskathedern gibt es im wesentlichen zwei Ausführungsformen. Bei beiden sind die zwei elektrischen Leitungen Drahtwedeln 30, $30^1$, 36, $36^1$, die in der einen Ausführungsform (Figur 3) parallel nebeneinander liegen und in der anderen Form (Figur 4) konzentrisch zueinander angeordnet sind.

In der Ausführung als kombinierter Mess- und Stimulationskatheder dienen beide Drahtwendeln 30 oder $30^1$ und 36 oder $36^1$ sowohl als Zuleitung zur Stimulationselektrode E als auch als Stromzuführung zur Messonde M. Der Kontakt zur Messonde M geschieht über das Ringelement 31, dessen Innengewinde für einen dauerhaften Druckkontakt sorgt. Das Ringelement 31 dient gleichzeitig als Träger für das (die) Sendeelement(e) 32, – jeweils eine rotstrahlende Licht emittierende Diode –, das (die) kathodenseitig mit dem Ringelement 31 kontaktiert (Klebekontakt) ist (sind). Das Ringelement 31 ist weiterhin mit der ebenfalls zur Stimulationselektrode führenden Drahtwendel 33 bzw. $33^1$ über Gewindekontakt elektrisch verbunden. Die Drahtwendel 33 ist zur Stimulationssicherung redundant ausgeführt. Zwischen dem steckerseitigen Teil des 1. Ringelementes 31 und dem 2. Ringelement 34 bzw. $34^1$ ist eine festklebende Ringisolierung 35 bzw. $35^1$ angebracht. Das 2. Ringelement 34 hat Gewindekontakt mit der Sondenleitung – Drahtwendel 36 bzw. $36^1$ und dient als Träger für das (die) Empfangselement(e) 37 – jeweils ein Phototransistor. Sofern die in den Figuren 2 und 3 nicht dargestellte Überbrückungsdiode $D_0$ für den Fall eines Leitungsbruches nicht im Empfangs- bzw. Sendeelement integriert ist, muss sie zusätzlich als Einzelelement auf einem der beiden Ringelemente 31 oder 34 angebracht werden. Die zweite Zuleitung zu den Sende- und Empfangselementen 32 und 37 (bzw. Überbrückungsdiode) ist jeweils ein Bonddraht 38 vom gegenüberliegenden Ringelement.

Um den Sondenkörper aus den beiden Ringelementen 31 und 34 ist als Schutz für die Sende- und Empfangselemente ein Glasring 39 gelegt, der an den Rändern fest mit den Ringelementen verschweisst ist.

Zur Isolierung zwischen den Zuleitungen (Drahtwendeln) dient der Isolierschlauch 40, nach aussen gegenüber dem Medium (Blut) dagegen der äussere transparente Isolierschlauch 41.

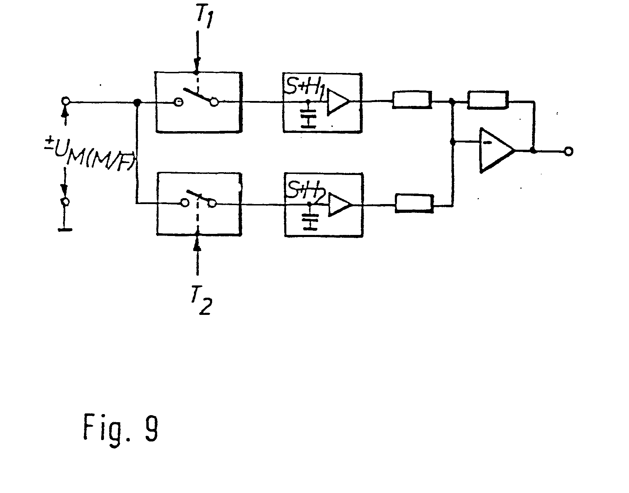

Figur 7 zeigt die Schaltung der Messonde bestehend aus dem Sendeelement 32 – einer Leuchtdiode –, dem Empfangselement 37 – einem npn-Phototransistor – und der Überbrückungsdiode $D_0$.

In Figur 8 ist das Funktionsprinzip der Messonde M anhand ihrer Strom-Spannungskennlinien 42 und 43 zu erkennen. Fehlt ein Reflexionskörper 44 in der Messanordnung Figur 7, dann ergibt dich die I-U-Kennlinie 42. Trifft jedoch Licht von einem Reflexionskörper 44 (im Anwendungsfall Blut) auf das Empfangselement 37, so ergibt sich die I-U-Kennlinie 43.

Die Intensität des reflektierten Lichtes ist somit bei konstantem Strom $J_K$ proportional der Änderung der Spannung $\Delta U_s$ und entsprechend bei konstanter Spannung $U_K$ proprotional der Stromänderung $\Delta J_s$.

Die angegebene Kombination von Sende- und Empfangselement behält auch bei Parallelschaltung mit ein oder zwei weiteren Kombinationen ihre prinzipielle Funktionsweise.

Figur 6 zeigt das Ausführungsbeispiel einer Ansteuerschaltung der Messonde, die einen Impuls mit konstantem Spannungsverlauf an den Zuleitungen 30, 36 zur Messonde erzeugt, so dass der Verlauf des Stromes $J_s$ durch die Messonde abhängig wird von dem durch die Messonde aufgenommenen Reflexionslicht und damit auch der Spannungsverlauf am Arbeitswiderstand $R_v$. Erreicht also die Sondenspannung $U_s$ einen festen Wert $U_K$ ist in diesem Moment die Messspannung $U_M$:

$$U_M = I_s \cdot R_v$$

und damit proportional zur Intensität des von der Messonde aufgenommenen, vom Blut reflektierten Lichts, wobei der Reflexionsfaktor des Blutes je nach Wellenlänge eine Funktion der Blutsauerstoffsättigung ist.

In Figur 9 ist das Funktionsprinzip für eine analoge Signal-Wandler-Schaltung 8 dargestellt. Damit wird das aus einem positiven, in der Taktphase $T_1$ ankommenden Messimpuls $U_{MM}$ und einem negativen, in der Taktphase $T_2$ ankommenden Fehlererkennungsimpuls $-U_{MF}$ bestehende Messignal $U_M$ von zwei Sample-and-Hold-Schaltungen $(S+H)_{1(2)}$ aufgenommen, so dass der eine Speicher $(S+H)_1$ über den in der Taktphase $T_1$ gechlossenen Schalter $S_1$ die Amplitude des Messimpulses $U_{MM}$ und der andere Speicher $(S+H)_2$ über den in der Taktphase $T_2$ geschlossenen Schalter $S_2$ die Amplitude des Fehlererkennungsimpulses $-U_{MF}$ speichert. In dem anschliessenden Summationsschalterkreis werden die Signalwerte $U_{MM}$ und $-U_{MF}$ derart ausgewertet, dass der im Messimpuls mitenthaltene, vor allem durch Widerstandsänderungen auf den Übertragungsleitungen 30, 36 und Temperaturdrift der optischen Messonde M entstandene Messfehler beseitigt wird.

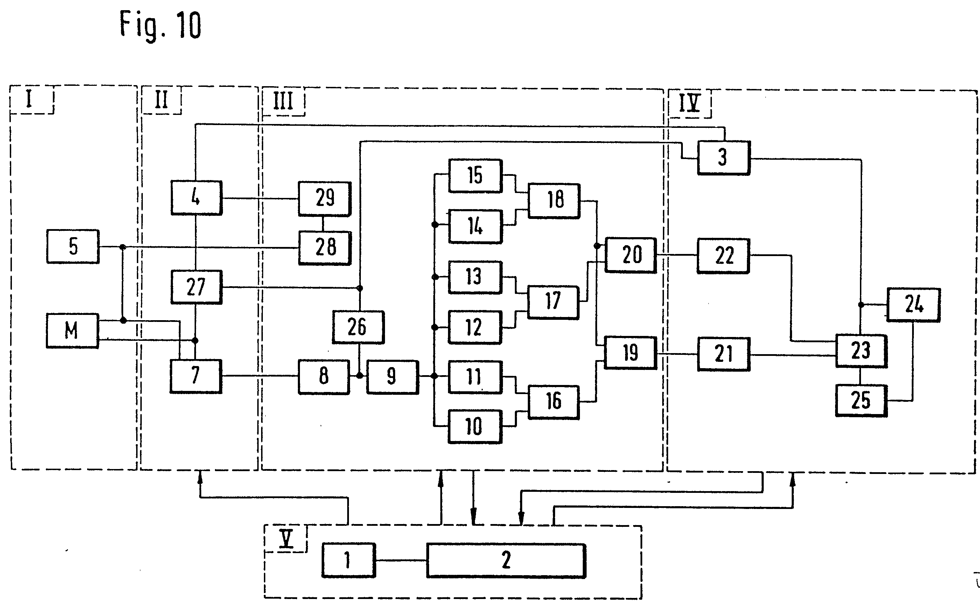

In Figur 10 sind als Blockschaltbild die wichtigsten Funktionen des erfindungsgemässen Herzschrittmachers dargestellt. Dabei kennzeichnen die gestrichelt eingerahmten Felder die übergeordneten Funktionseinheiten und zwar bedeuten:

I     Katheder
II    Katheder-Ansteuerung
III   Messwert-Auswertung
IV   Frequenzregelung
V    Programmsteuerung

Es liefert ein festfrequenter Taktgenerator 1 die Zeitbasis für eine Programmsteuerung 2, die alle Mess- und Regelvorgänge der Schrittmacherschaltung steuert.

Die Programmsteuerung startet in Abhängigkeit von einem Stimulationsfrequenzgeber 3 vorgegebenen Zeitcode die Aussendung des Stimulationsimpulses durch einen Stimulationspulsgeber 4 über eine Stimulationselektrode 5 sowie direkt anschliessend die Messung der Blutsauerstoffsättigung über die Messonde M mit der Messonden-Ansteuerung 7. Das Messignal wird im Signalwandler 8 von Störgrössen getrennt, verstärkt und bei Einsatz einer digitalen Messwertverarbeitung in digitale Form umgewandelt. Ein Integrator 9 bildet den Mittelwert der Messignale über einen vorgegebenen Zeitraum. Die Speicher 10 bis 15 übernehmen den integrierten Signalwert, und zwar die Speicher 10 und 11 abwechselnd im Zeitraum $\Delta t_1$ und die Speicher 12 und 13 abwechselnd im Zeitraum $\Delta t_4$. Im Maximalwertspeicher 14 wird der in einem vorgegebenen Zeitbereich $\Delta t_0$ höchste eintreffende Messignalwert gespeichert und im Minimalwertspeicher 15 der jeweils niedrigste Wert.

Im Differenzbildner 16 wird die Messwertdifferenz $\Delta S_{02}$ zwischen dem neuen und dem jeweils vorhergehenden Messignal in Speicher 10 bzw. Speicher 11 festgestellt und im Differenzbildner 17 die Messwertdifferenz zwischen Inhalt von Speicher 12 bzw. 13. Im Differenzbildner 18 wird der maximale Messwertschwankungsbereich $S_{02\,max} - S_{02\,min} = \Delta S_{02max}$, so dass das im Dividierer 19 und 20 ermittelte Verhältnis von kurzzeitiger Messwertschwankung zum maximalen Schwankungsbereich jeweils die Regelgrösse $B_s$ ergibt:

$$B_{S1} = \frac{\Delta S_{02}\,(t_1) \cdot \Delta t_0}{\Delta S_{02}\,max \cdot \Delta t_1}$$

In der Frequenzregelung IV stellt ein Komparator 21 fest, ob die zeitliche Änderung der normierten Messgrösse, d.h. die erste Regelgrösse $B_{S1}$ grösser oder kleiner als ein vorgegebener erster Grenzwert $+A_1$ bzw. $-A_1$ ist.

In der folgenden Stimulationsfrequenzsteuerung 23 wird im Fall $B_{S1} < A_1$ eine Änderung der Stimulationsfrequenz um einen positiven Wert $+\Delta f$ und im Fall $B_{S1} > +A_1$ um einen negativen Wert $-\Delta f$ bewirkt und das Vorzeichen der Änderung im Speicher 24 abgespeichert. Im Fall $-A_1 < B_{S1} < +A_1$ wird nach einem fest vorgegebenen Zeitintervall $\Delta t_5$ automatisch eine Änderung der Stimmulationsfrequenz in der Frequenzsteuerung 23 veranlasst, wobei das Vorzeichen der Änderung entgegengesetzt dem im Speicher 24 festgehaltenen Wert ist, solange die Tendenzsteuerung 25 keine Vorzeichen-Wiederhohlung bewirkt. Nach dem vorgegebenen Zeitintervall $\Delta t_4$ beurteilt der Komparator 22, ob die Änderung der Messgrösse d.h. die zweite Regelgrösse $B_{S4}$ grösser als ein fester zweiter Grenzwert $A_2$ bzw. kleiner als $-A_2$ ist, worauf die vorausgegangene Frequenzänderung entweder rückgängig gemacht wird oder bleibt.

Die Fehlererkennung 26 vergleicht das von der Messauswertung aufgenommene Signal mit zulässigen Grenzwerten und setzt bei Überschreitung dieser Grenzwerte den Stimulationsfrequenzgeber 3 auf Festfrequenz $f_0$ und schliesst über den Schalter 27 die beiden Kathederanschlüsse kurz, wodurch die Messung und Regelung ausser Kraft gesetzt wird.

Im EKG-Verstärker 28 wird die Herzeigenaktivität zwischen den Stimulationen überwacht und über den Komperator 29 im Fall einer Herzeigenerregung die Stimulation durch den Impulsgeber verhindert.

Figur 10 zeigt die erfindungsgemässe Regelung der Herzschrittmacher-Stimulationsfrequenz f in Abhängigkeit von der Belastung des Patienten, dargestellt durch den Zusammenhang im zeitlichen Verlauf des Messwertes der Blutsauerstoffsättigung $S_{02}$, dessen Veränderung pro Zeiteinheit $\Delta t_1$ und $\Delta t_4$, sowie der dadurch bewirkten Änderung der Frequenz f.

Bei Beginn der Belastungsphase BP sinkt die zentralvenöse Sauerstoffsättigung $S_{02}$, d.h. die Änderung pro Zeiteinheit $\Delta t_1$ bezogen auf eine maximale Schwankungsbreite $\Delta S_{02max}/\Delta t_0$ zwischen den Grenzwerten $S_{02max}$ und $S_{02min}$ ergibt einen negativen Wert für $B_{S1}$. Ist dieser kleiner als $-A_1$ folgt innerhalb des Zeitintervalls $\Delta t_2$ automatisch eine Frequenzänderung um $+\Delta f_1$. Erreicht dagegen die Sauerstoffsättigung $S_{02}$ in der Belastungsphase BP einen gewissen Gleichgewichtszustand, so dass der Wert von $B_{S1}$ zwischen $-A_1$ und $+A_1$ pendelt, beginnt die Optimalregelung, und zwar im Sinne einer besseren Sauerstoffversorgung zuerst immer mit einer positiven Frequenzänderung $+\Delta f_2$ nach $\Delta t_5$. Bewirkt dieses $+\Delta f_2$ eine Erhöhung des $S_{02}$-Wertes innerhalb der Zeiteinheit $\Delta t_4$ und ist dieser Wert – bezogen wieder auf $\Delta S_{02max}/\Delta t_4$ – grösser als ein Festwert $+A_2$, dann bleibt die Frequenzänderung und veranlasst aufgrund des positiven Ergebnisses zugleich nach weiteren $\Delta t_5$ eine erneute Frequenzerhöhung um $\Delta f_2$. Ergibt diese keine positive $S_{02}$-Änderung, ist also nach $\Delta t_4$ der $B_{S4}$-Wert kleiner als $+A_2$, wird die Frequenzänderung rückgängig gemacht. Beginnt dann die Ruhephase RP, und steigt der $B_{S1}$-Wert über den Festwert $+A_1$, so folgt automatisch eine negative Frequenzänderung $-\Delta t_2$ solange, bis $B_{S1}$ wieder kleiner ist als $+A_1$. Dann setzt erneut die Optimalregelung mit einer positiven Frequenzänderung $+\Delta f_2$ nach $\Delta t_5$ ein und wiederholt dies so oft, wie nach $\Delta t_4$ der $B_{S4}$-Wert grösser ist als $+A_2$, also eine Verbesserung der Sauerstoffsättigung $S_{02}$ erfolgt.

## Patentansprüche

1. Einrichtung zur Frequenzregelung eines Herzschrittmachers in Abhängigkeit von der gemessenen Änderung der zentralvenösen Blutsauerstoffsättigung, dadurch gekennzeichnet, dass

a) zur Messung der zentralvenösen Sauerstoffsättigung eine mit dem Herzschrittmacher verbindbare optische Messonde (M) mit einem Lichtsendeelement (32) vorgesehen ist, dessen ausgesendetes und vom Blut (44) reflektiertes Licht in einem in der Messonde angeordneten Lichtempfangselement (37) ein elektrisches Signal verursacht, das bei konstant vorgegebener Sondenspannung ($U_K$) in einer Erhöhung ($\Delta I_s$) des Stromflusses ($I_s$) durch die Sonde oder bei konstant vorgegebenem Stromfluss ($I_K$) in einer Dämpfung ($\Delta U_S$) der Sondenspannung ($U_S$) besteht,

b) Mittel (10, 11, 16; 14, 15, 18, 21) zur Bestimmung einer ersten Regelgrösse ($B_{S1}$) in Form des Quotienten

$$B_{S1} = \frac{\Delta S_{02}(t_1) \cdot \Delta t_0}{\Delta S_{02} \max \cdot \Delta t_1}$$

vorgesehen sind, der aus der Änderung des durch die Lichtreflektion verursachten elektrischen Signals der Sonde ($\Delta S_{02}(t_1)$) innerhalb erster, vorgegebener Zeitabstände ($\Delta t_1$) (Sekunden, Minuten) dividiert durch die maximale Änderung des Signals ($\Delta S_{02\ max}$) innerhalb eines in bezug auf die ersten Zeitabstände langer Zeitraum ($\Delta t_0$) (Stunden, Tage) besteht,

c) eine Nachlaufregelung (21, 23, 24) vorgesehen ist, die jeweils im Anschluss an den ersten Zeitabstand ($\Delta t_1$) innerhalb eines vorgegebenen zweiten Zeitabstandes ($\Delta t_2$) die Stimulationsfrequenz um einen vorgebbaren konstanten ersten Betrag ($\Delta f_1$) erhöht, bzw. erniedrigt, wenn die erste Regelgrösse einen negativen bzw. positiven Wert aufweist, und ihr Betrag ($|B_{S1}|$) einen vorgegebenen ersten Grenzwert ($A_1$) übersteigt, wobei die Frequenzänderung nur solange erfolgt, bis ein vorgegebener Grenzwert $f_{min}$ bzw. $f_{max}$ erreicht ist.

2. Einrichtung nach Anspruch 1, dadurch gekennzeichnet, dass eine zweite, optimierende Regelung der Stimulationsfrequenz f vorgesehen ist, die die Mittel (12, 13, 17; 14, 15, 18, 20) zur Bestimmung einer zweiten Regelgrösse ($B_{S4}$) in Form des Quotienten

$$B_{S4} = \frac{\Delta S_{02}(t_4) \cdot \Delta t_0}{\Delta S_{02\ max} \cdot \Delta t_4}$$

umfasst, der aus der Änderung des Signals der Sonde ($\Delta S_{02}(t_4)$) innerhalb vorgegebener vierter Zeitabständen ($\Delta t_4$) dividiert durch die maximale Änderung des Signals ($S_{02max}$) innerhalb des langen Zeitraums ($\Delta t_0$) besteht, und die Mittel (22, 23, 24) aufweist, die jeweils im Anschluss an vorgegebene fünfte Zeitabschnitte ($\Delta t_5$), in denen der Betrag der ersten Regelgrösse ($B_{S1}$) kleiner als der des ersten Grenzwertes ($A_1$) bleibt (also: $|B_{s1}| < A_1$) selbständig innerhalb vorgegebener dritter Zeitabstände ($\Delta t_3$) eine Erhöhung bzw. Erniedrigung der Stimulationsfrequenz um einen vorgebbaren konstanten zweiten Betrag ($\Delta f_2$) bewirkt und die nach einem weiteren vierten Zeitintervall ($\Delta t_4$) die zweite Regelgrösse ($B_{S4}$) dahingehend auswerten, dass eine Frequenzerhöhung nur dann bestehen bleibt, wenn sie eine Vergrösserung der zweiten Regelgrösse ($B_{S4}$) über den Wert eines zweiten Grenzwertes ($A_2$) hinaus bewirkt hat, und dass eine Frequenzerniedrigung immer dann bestehen bleibt, wenn sie keine Verkleinerung der zweiten Regelgrösse (B_{S4}) über den negativen Wert des zweiten Grenzwertes (A_2) hinaus bewirkt hat.

3. Einrichtung nach Anspruch 2, dadurch gekennzeichnet, dass die optimierende Regelung der Stimulationsfrequenz eine Tendenzsteuerung (25) zu einer Festfrequenz $f_0$ aufweist, die dadurch erzielt wird, dass die selbständige Frequenzänderung um den zweiten Betrag ($\Delta f_2$) häufiger negativ ist, wenn die Stimulationsfrequenz grösser ist als ein vorgegebener Festwert ($f_0$) und dass sie häufiger positiv ist, wenn die Stimulationsfrequenz kleiner ist als der Festwert ($f_0$).

4. Einrichtung nach Anspruch 2 oder 3, dadurch gekennzeichnet, dass im Fall $| B_{S1} | > A_1$ eine Kontrollstufe vorgesehen ist, die feststellt, ob im fünften Zeitabstand $\Delta t_5$ vorher eine selbständige Frequenzänderung um den zweiten Betrag ($\Delta f_2$) stattgefunden hat, und welche Vorzeichen diese Frequenzänderung hatte, und die diese Frequenzänderung rückgängig macht, wenn sie der durch die Nachlaufregelung vorgesehenen Antwort auf $| B_{S1} | > A_1$ entgegengesetzt ist, wodurch die Kontrollstufe die Dominanz der Nachlaufregelung gegenüber der Optimalregelung sichert.

5. Einrichtung nach Anspruch 1, dadurch gekennzeichnet, dass eine Programmsteuerung (V) vorgesehen ist, die die Messwerterfassung und -auswertung direkt vor oder nach Aussenden des Stimulationsimpulses bzw. nach Empfang des Erkennungssignals bei einer Herzeigenerregung startet.

6. Einrichtung nach Anspruch 1, dadurch gekennzeichnet, dass Fehlererkennungsmittel (26) vorgesehen sind, die einen Fehlerfall bei der Messwerterfassung, der Messwertauswertung und der Frequenzregelung durch Grenzwertvergleich erkennen, und die in jedem Fehlerfall die Stimulationsfrequenz (f) auf einen Festwert ($f_0$) umschalten und die zwei Zuleitungen (30, 36) zur Messonde für die Stimulation galvanisch überbrücken.

7. Einrichtung nach Anspruch 1, dadurch gekennzeichnet, dass

a) die optische Messonde (M) mindestens eine Kombination von nur zwei aktiven optoelektronischen Bauelementen (32, 37), nämlich einem Lichtsendeelement (32) und einem Lichtempfangselement (37), sowie zusätzlich ein Halbleiterelement (D_0) enthält;

b) dass die optische Messonde (M) im Stimulationskatheder (K) miteingebaut ist;

c) dass die optische Messonde (M) über vorzugsweise nur zwei elektrische Leitungen (30, 36; 30^1, 36^1) die im Stimulationskatheder geführt werden mit einer Ansteuerschaltung (7) verbunden ist;

d) dass die Ansteuerschaltung (7) mit Hilfe der Messonde (M) ein Messignal ($U_M$) erzeugt;

e) dass die Signalwandlerschaltung (8) die mit der optischen Messonde (M) und der Ansteuerschaltung (7) gewonnenen Messignale ($U_M$) von Störgrössen trennt, verstärkt und in eine für die (digitale) Frequenzregelung geeignete Signalform umwandelt.

8. Einrichtung nach Anspruch 7, dadurch gekennzeichnet, dass in der Messonde das Lichtsendeelement (32) – eine Licht emittierende Diode (LED) – und das Lichtempfangselement (37) – ein Phototransistor – derart parallel geschaltet sind, dass bei Verwendung eines npn (pnp)-Phototransistors die Kathode der Diode (LED) mit dem Emitter (Kollektor) des Phototransistors und die Anode der Diode (LED) mit dem Kollektor (Emitter) des Phototransistors verbunden sind.

9. Einrichtung nach Anspruch 7, dadurch gekennzeichnet, dass in der Messonde (M) eine Halbleiterdiode (D_0) derart integriert ist, dass die Kathode der Diode (D_0) mit der Anode der Licht emittierenden Diode (32) und die Anode der Diode (D_0) mit der Kathode der Diode (32) verbunden ist.

10. Einrichtung nach einem der Ansprüche 7 bis 9, dadurch gekennzeichnet, dass die Ansteuerschaltung der Messonde (M) durch einen elektrischen Impulsgenerator gebildet ist, der vorzugsweise einen positiven und negativen Impuls der gesteuerten Sondenspannung ($U_K$) bzw. des Sondenstromes ($I_K$) erzeugt, der über mindestens einen Arbeitswiderstand ($R_v$) derart an die Zuleitungen (30, 36) zur Messonde (M) weitergegeben wird, dass die Spannung ($U_M$) an dem bzw. den Widerständen ($R_v$) als Messwert ausnutzbar ist.

11. Einrichtung nach Anspruch 10, dadurch gekennzeichnet, dass die Signal-Wandler-Schaltung (8), die am Arbeitswiderstand ($R_v$) sequentiell anliegend positiven und negativen Impulse ($+ U_{MM}, - U_{MF}$) derart verarbeitet, dass die im eigentlichen Messimpuls ($U_{MM}$) mitenthaltenen Messfehler vor allem durch Widerstandänderungen der Zuleitungen (30, 36) und Temperaturdrift der Messonde (M) beseitigt werden.

12. Einrichtung nach einem der Ansprüche 7 bis 11, dadurch gekennzeichnet, dass der Körper der Messonde (M) aus zwei gegeneinander isolierten metallischen Ringelementen (31, 34) zusammengesetzt ist, die als Träger mindestens eines Sende- (32) bzw. Empfangselementes (37) dienen und zugleich elektrisches Verbindungsstück zu den Sondenleitungen (36, 30) sind.

13. Einrichtung nach Anspruch 12, dadurch gekennzeichnet, dass mehrere Lichtsende- (32) und Lichtempfangselemente (37) derart kreisförmig um die Achse des Katheders auf den Ringelementen (31, 34) angeordnet sind, dass der Messwinkel in der Querschnittsebene des Katheders bis zu 360° betragen kann.

14. Einrichtung nach einem der Ansprüche 7 bis 13, dadurch gekennzeichnet, dass beide elektrische Zuleitungen (30, 36) zur Messonde (M) zugleich als Stromzuführung zur Stimulationselektrode (E) dienen.

15. Einrichtung nach Anspruch 7, dadurch gekennzeichnet, dass ein transparenter Schutzmantel (39), vorzugsweise aus Glas, die Messonde (M) umgibt.

16. Einrichtung nach Anspruch 7, dadurch gekennzeichnet, dass die Messonde (M) zwischen der als Drahtwendel ausgebildeten elektrischen Stimulationsleitung (30) und dem transparenten Isolierschlauch (41) derart angeordnet ist, dass dieser weder unterbrochen, noch wesentlich verformt wird.

17. Einrichtung nach Anspruch 7, dadurch gekennzeichnet, dass die Messonde (M) vorzugsweise derart im Katheder (K) integriert wird, dass sie bei Implantation im Bereich der Segelklappen positioniert werden kann, also etwa 4 bis 8 cm hinter der Stimulationselektrode.

## Revendications

1. Dispositif pour la régulation de la fréquence de stimulation d'un entraîneur cardiaque en fonction de la modification mesurée de la saturation en oxygène du sang des veines centrales caractérisé

a) en ce que pour la mesure de la saturation en oxygène du sang des veines centrales, il est prévu une sonde de mesure optique (M) qui peut être reliée à l'entraîneur cardiaque et qui est accompagnée d'un élément émetteur de lumière (32), dont la lumière émise, qui est réfléchie par le sang (44), provoque, dans un élément récepteur de lumière (37), qui est adjoint à la sonde de mesure, un signal électrique qui consiste, dans le cas d'une tension ($U_K$) à la sonde donnée constante, en une augmentation ($\Delta I_S$) du flux de courant ($I_S$) par la sonde ou, dans le cas d'un flux de courant ($I_K$) donné constant, en une atténuation ($\Delta U_S$) de la tension ($U_S$) à la sonde,

b) en ce qu'il est prévu des moyens (10, 11, 16; 14, 15, 18, 21) destinés à la détermination d'une première grandeur de régulation ($B_{S1}$) se présentant sous la forme du quotient

$$B_{S1} = \frac{\Delta S_{02}(t_1) \cdot \Delta t_0}{\Delta S_{02} \max \cdot \Delta t_1}$$

quotient qui est obtenu par la division de la modification du signal électrique de la sonde [$\Delta S_{02}(t_1)$], provoqué par la réflexion de la lumière, dans de premiers écarts de temps donnés ($\Delta t_1$) (secondes, minutes), par la modification maximale du signal ($\Delta S_{02 \max}$), dans un espace de temps ($\Delta t_0$) (heures, jours) plus long que les premiers écarts de temps,

c) en ce qu'il est prévu une régulation de poursuite (21, 23, 24) qui, à la suite du premier écart de temps donné ($\Delta t_1$), dans un deuxième écart de temps donné ($\Delta t_2$) augmente ou abaisse la fréquence de stimulation d'une première valeur constante pouvant être donnée ($\Delta f_1$), si la première grandeur de régulation présente une valeur négative ou une valeur positive, et en ce que sa valeur ($B_{S1}$) dépasse une première valeur limite donnée ($A_1$), la modification de la fréquence n'ayant lieu que pendant la durée à l'expiration de laquelle une valeur limite donnée $f_{min}$ ou $f_{max}$ est atteinte.

2. Dispositif suivant la revendication 1, caractérisé en ce qu'il est prévu une deuxième régulation d'optimisation de la fréquence de stimulation f comportant les moyens (12, 13, 17; 14, 15, 18;

20) destinés à la détermination d'une deuxième grandeur de régulation ($B_{S4}$) se présentant sous la forme du quotient

$$B_{S4} = \frac{\Delta S_{02}(t_4) \cdot \Delta t_0}{\Delta S_{02 \max} \cdot \Delta t_4}$$

quotient qui est obtenu par la division de la modification du signal de la sonde [$\Delta S_{02}(t_4)$], dans de quatrièmes écarts de temps donnés ($\Delta t_4$) par la modification maximale du signal ($S_{02 \max}$), dans le long espace de temps ($\Delta t_0$), et comportant les moyens (22, 23, 24) qui, à la suite de cinquièmes laps de temps donnés ($\Delta t_5$), au cours desquels la valeur de la première grandeur de régulation ($B_{S1}$) reste plus faible que celle de la première grandeur limite ($A_1$) (c'est-à-dire au cours desquels $|B_{S1}| < A_1$), déterminent indépendamment, dans de troisièmes écarts de temps donnés ($\Delta t_3$), une augmentation ou un abaissement de la fréquence de stimulation d'une deuxieme valeur constante pouvant être donnée ($\Delta f_2$), et qui, après un autre quatrième intervalle de temps ($\Delta t_4$), évaluent la deuxième grandeur de régulation ($B_{S4}$) en partant de ce qu'une augmentation de la fréquence ne subsiste que si elle a déterminé un accroissement de la deuxième grandeur de régulation ($B_{S4}$) au-delà de la valeur d'une deuxième valeur limite ($A_2$) et de ce qu'un abaissement de la fréquence susbiste toujours s'il n'a pas déterminé de diminution de la deuxième valeur de régulation ($B_{S4}$) au-delà de la valeur négative de la deuxième valeur limite ($A_2$).

3. Dispositif suivant la revendication 2, caractérisé en ce que la régulation d'optimisation de la frequence de stimulation comporte une commande tendantielle (25) vers une fréquence $f_0$ qui est obtenue par le fait que la modification de fréquence indépendante de la deuxiéme valeur ($\Delta f_2$) est plus souvent négative si la fréquence de stimulation est supérieure à une valeur fixe donnée ($f_0$) et par le fait que cette modification de fréquence indépendante de la deuxième valeur ($\Delta f_2$) est plus souvent positive si la fréquence de stimulation est inférieure à la valeur fixe ($f_0$).

4. Dispositif suivant l'une ou l'autre des revendications 2 et 3, caractérisé en ce que pour le cas où $|B_{S1}| > A_1$, il est prévu un étage de contrôle qui établi si au cours du cinquième écart de temps $\Delta t_5$, une modification de fréquence indépendante de la deuxième valeur ($\Delta f_2$) a au préalable eu lieu et quel signe avait cette modification de fréquence, et qui rend cette modification de fréquence régressive si elle est opposée à la réponse à $|B_{S1}| > A_1$ prévue par la régulation par poursuite, d'où l'étage de contrôle assure la dominance de la régulation par poursuite par rapport à la régulation optimale.

5. Dispositif suivant la revendication 1, caractérisé en ce qu'il est prévu une commande par programme (V) qui fait démarrer le relèvement de la valeur de mesure et l'évaluation de cette valeur de mesure directement avant ou après l'émission de l'impulsion de stimulation ou après la réception du signal de reconnaissance ou identification, dans le cas d'une excitation propre au cœur.

6. Dispositif suivant la revendication 1, caractérisé en ce qu'il est prévu des moyens de reconnaissance ou identification d'erreurs (26) qui reconnaissent ou identifient par comparaison de la valeur limite un cas d'erreur lors du relèvement de la valeur de mesure, lors de l'évaluation de la valeur de mesure et lors de la régulation de fréquence, et qui, dans chaque cas d'erreur, font passer la fréquence de stimulation (f) à une valeur ($f_0$) et pontent galvaniquement les deux conducteurs (30 et 36) allant à la sonde de mesure, pour la stimulation.

7. Dispositif suivant la revendication 1, caractérisé

a) en ce que la sonde de musure optique (M) comporte au moins une combinaison de deux éléments constitutifs opto-électroniques actifs seulement (32 et 37), qui sont un élément émetteur de lumière (32) et un élément récepteur de lumière (37), ainsi qu'additionnellement, un élément semi-conducteur ($D_0$);

b) en ce que la sonde de mesure optique (M) est incorporée au cathéter de stimulation (K);

c) en ce que la sonde optique (M) est reliée à un montage d'attaque (7) avantageusement par l'intermédiaire de deux conducteurs électriques (30, 36; $30^1$, $36^1$) seulement, qui sont guidés dans le cathéter de stimulation;

d) en ce que la montage d'attaque (7) produit, à l'aide de la sonde de mesure (M), un signal de mesure ($U_M$);

e) en ce que le montage convertisseur de signaux (8) sépare des grandeurs perturbatrices les signaux de mesure ($U_M$) obtenus à l'aide de la sonde de mesure optique (M), et du montage d'attaque (7), amplifie ces signaux de mesure obtenus à l'aide de la sonde de mesure optique (M) et du montage d'attaque (7) et les convertit en une forme de signaux qui convient pour la régulation (digitale) de la fréquence.

8. Dispositif suivant la revendication 7, caractérisé en ce que dans la sonde de mesure, l'élément émetteur de lumière (32) – une diode émettrice de lumière (LED) – et l'élément récepteur de lumière (37) – un phototransistor – sont montés en parallèle de telle sorte que, dans le cas où il est utilisé un phototransistor npn (pnp), la cathode de la diode (LED) soit reliée à l'émetteur (collecteur) du phototransistor et que l'anode de la diode (LED) soit reliée au collecteur (émetteur) du phototransistor.

9. Dispositif suivant la revendication 7, caractérisé en ce qu'à la sonde de mesure (M), il est incorporé une diode semi-conductrice ($D_o$) de telle sorte que la cathode de la diode ($D_o$) soit relié à l'anode de la diode émettrice de lumière (32) et que l'anode de la diode ($D_o$) soit relié à la cathode de la diode (32).

10. Dispositif suivant l'une quelconque des revendications 7 à 9, caractérisé en ce que le montage d'attaque de la sonde de mesure (M) est constitué par un générateur d'impulsions électriques qui produit avantageusement une impulsion positive et une impulsion négative de la tension commandée ($U_K$) de la sonde et du courant ($I_K$)

de la sonde, qui, par l'intermédiaire d'au moins une résistance de travail ($R_v$), est transmise aux conducteurs (30 et 36) allant à la sonde de mesure (M) de telle sorte que la tension ($U_M$) à la résistance ou aux résistances ($R_v$) puisse être utilisée comme valeur de mesure.

11. Dispositif suivant la revendication 10, caractérisé en ce que le montage convertisseur de signaux (8) traite les impulsions positives et les impulsions négatives ($+U_{MM}$, $-U_{MF}$) appliquées en séquence à la résistance de travail ($R_v$) de telle sorte que les erreurs de mesure contenues dans l'impulsion de mesure proprement dite ($U_{MM}$) soient avant tout écartées par les modifications de résistance des conducteurs (30 et 36) et par l'écart de température de la sonde de mesure (M).

12. Dispositif suivant l'une quelconque des revendication 7 à 11, caractérisé en ce que le corps de la sonde de mesure (M) est composé de deux éléments annulaires métalliques (31 et 34) qui sont isolés l'un par rapport à l'autre, éléments annulaires qui servent de supports à au moins un élément émetteur (32) et un élément récepteur (37) et qui constituent en même temps un moyen de liaison électrique avec les conducteura (36 et 30) de la sonde.

13. Dispositif suivant la revendication 12, caractérisé en ce que plusieurs éléments émetteurs de lumière (32) et plusieurs éléments récepteurs de lumière (37) sont disposés en cercle autour de l'axe du cathéter, sur les éléments annulaires (31 et 34), de telle sorte que l'angle de mesure, dans le plan de la section transversale du cathéter, puisse être d'une valeur allant jusqu'à 360°.

14. Dispositif suivant l'une quelconque des revendications 7 à 13, caractérisé en ce que les deux conducteurs électriques (30 et 36) allant à la sonde de mesure (M) servent en même temps de conducteurs d'amenée de courant à l'électrode de stimulation (E).

15. Dispositif suivant la revendication 7, caractérisé en ce qu'une enveloppe de protection transparente (39), avantageusement en verre, entoure la sonde de mesure (M).

16. Dispositif siuvant la revendication 7, caractérisé en ce que la sonde de mesure (M) est prévue entre le conducteur de stimulation électrique (30), qui se présente sous la forme d'un enroulement de fil, et le conduit flexible transparent d'isolation (41) de telle sorte que ce conduit flexible ne soit ni interrompu, ni déformé dans une mesure importante.

17. Dispositif suivant la revendication 7, caractérisé en ce que la sonde de mesure (M) est avantageusement incorporée au cathéter (K) de telle façon qu'elle puisse être positionnée dans la zone des valvules lors de l'implantation, c'est-à-dire à peu près à 4 à 8 cm à l'arrière de l'électrode de stimulation.

**Claims**

1. A device for regulating the stimulation rate of cardiac pacemakers in dependence on the measured change of the central venous blood oxygen saturation, characterized in that

a) for the measurement of the central venous blood oxygen saturation an optical measuring probe (M) is provided which is connectible to the cardiac pacemaker and has a light-transmitting element (32), the light transmitted thereby and reflected by the blood (44) causing an electrical signal in a light-receiving element (37) arranged in the measuring probe, the electrical signal consisting in an increase ($\Delta I_S$) of the current flow ($I_S$) through the measuring probe at a constant prescribed probe voltage ($U_K$) or in a damping ($\Delta U_S$) of the probe voltage ($U_S$) at a constant prescribed current flow ($I_K$),

b) means (10, 11, 16; 14, 15, 18, 21) are provided for determining a first controlled variable ($B_{S1}$) in the form of the quotient

$$B_{S1} = \frac{\Delta S_{02}(t_1) \cdot \Delta t_0}{\Delta S_{02}\text{max} \cdot \Delta t_1}$$

which consists of the change of the electrical signal of the probe ($\Delta S_{02}(t_1)$), caused by the light reflection, within first prescribed time intervals ($\Delta t_1$) seconds, minutes) divided by the maximum change of the signal ($\Delta S_{02max}$) within a period of time ($\Delta t_0$) (hours, days) which is long as compared with the first time intervals,

c) a follow-up control (21, 23, 24) is provided which, subsequent to the first time interval ($\Delta t_1$), increases or decreases the stimulation rate within a prescribed second time interval ($\Delta t_2$) by a prescribable constant first value ($\Delta f_1$) when the first controlled variable has a negative or a positive value, and its amount ($|B_{S1}|$) exceeds a prescribed first limiting value ($A_1$), the change in the rate only being effected until a prescribed limiting value $f_{min}$ or $f_{max}$ has been reached.

2. A device as claimed in claim 1, characterized in that a second optimizing regulation of the stimulation rate (f) is provided which comprises the means (12, 13, 17; 14, 15, 18; 20) for determining a second controlled variable ($B_{S4}$) in the form of the quotient

$$B_{S4} = \frac{\Delta S_{02}(t_4) \cdot \Delta t_0}{\Delta S_{02 \ max} \cdot \Delta t_4}$$

which consists of the change of the probe signal ($\Delta S_{02}(t_4)$) within prescribed fourth time intervals ($\Delta t_4$) divided by the maximum change of the signal ($\Delta S_{02max}$) within the long period of time ($\Delta t_0$), and which comprises means (22, 23, 24) which, subsequent to prescribed fifth time intervals ($\Delta t_5$) in which the amount of the first controlled variable ($B_{S1}$) remains smaller than that of the first limiting value ($A_1$) (thus: $|B_{S1}| < A_1$), independently cause an increase or decrease of the stimulation rate within prescribed third time intervals ($\Delta t_3$) by a prescribable constant second amount ($\Delta f_2$) and which, after a further fourth time interval ($\Delta t_4$), evaluate the second controlled variable ($B_{S4}$) to the effect that an increase in the rate is only retained if it has caused an increase of the second controlled variable ($B_{S4}$) beyond the value of a second limiting value ($A_2$) and that a decrease in the rate is always retained if it has not caused a decrease of the second controlled variable ($B_{S4}$) beyond the negative value of the second limiting value ($A_2$).

3. A device as claimed in claim 2, characterized in that the optimizing regulation of the stimulation rate has a tendency control (25) to a fixed stimulation rate ($f_0$) which is achieved by the independent change in the stimulation rate by the second amount ($\Delta f_2$) more frequently being negative if the stimulation rate is greater than a prescribed fixed value ($f_0$) and by more frequently being positive when the stimulation rate is smaller than the fixed value ($f_0$).

4. A device as claimed in claim 2 or 3, characterized in that in the case in which $|B_{S1}| > A_1$ a check stage is provided which determines whether an independent change in the stimulation rate by the second amount ($\Delta f_2$) has previously taken place in the fifth time interval ($\Delta t_5$) and which signs this change in the rate had, and which reverses this change in rate if it is opposed to the answer, provided by the follow-up control, to $|B_{S1}| > A_1$, the check stage therefore ensuring the dominance of the follow-up control over the optimum regulation.

5. A device as claimed in claim 1, characterized in that a programm control (V) is provided which starts the data acquisition and evaluation directly before or after the emission of the stimulation pulse, or after receiving the detection signal in the case of a self-excitation of the heart.

6. A device as claimed in claim 1, characterized in that error detection means (26) are provided which detect an error during the data acquisition, the data evaluation and the rate regulation by a commparison with limiting values and which, in each case of error, switch the stimulation rate (f) to a fixed value ($f_0$) and galvanically bridge the two supply lines (30, 36) to the measuring probe for the stimulation.

7. A device as claimed in claim 1, characterized in that

a) the optical measuring probe (M) contains at least one combination of only two active optoelectronic structural elements (32, 37), namely a lightemitting element (32) and a light-receiving element (37) as well as additionally a semiconductor element ($D_0$);

b) the optical measuring probe (M) is incorporated in the stimulation catheter (K);

c) the optical measuring probe (M) is connected via preferably only two electric lines (30, 36; $30^1$; $36^1$) leading through the stimulation catheter to a control circuit (7);

d) the control circuit (7) generates with the aid of the measuring probe (M) a measuring signal ($U_M$);

e) the measured signals ($U_M$) attained with the optical measuring probe (M) and the control circuit (7) are separated from interfering signals, amplified and converted into a signal form suitable for the (digital) rate regulation by the signal converting circuit (8).

8. A device as claimed in claim 7, characterized in that the light-emitting element (32) – a light-emitting diode (LED) – and the light-receiving element (37) – a phototransistor – are connected with one another in parallel, in the measuring probe, in such a manner that, when an npn (pnp) phototransistor is used, the cathode of the diode (LED) is connected with the emitter (collector) of the phototransistor and the anode of the diode (LED) with the collector (emitter) of the phototransistor.

9. A device as claimed in claim 7, characterized in that a semiconductor diode $(D_o)$ is integrated into the measuring probe (M) in such a manner that the cathode of the diode $(D_o)$ is connected with the anode of the light-emitting diode (32) and the anode of the diode $(D_o)$ is connected with the cathode of the diode (32).

10. A device as claimed in one of claims 7 to 9, characterized in that the control circuit of the measuring probe (M) is formed by an electrical pulse generator which preferably produces a positive and a negative pulse of the controlled probe voltage $(U_K)$ or probe current $(I_K)$ which is then passed on, via at least one load resistance $(R_v)$, to the supply lines (30, 36) to the measuring probe (M) in such a manner that the voltage $(U_M)$ at the resistance or resistances $(R_v)$ can be used as a measured value.

11. A device as claimed in claim 10, characterized in that the signal converting circuit (8) processes the positive and negative pulses ($+U_{MM}$, $-U_{MF}$) sequentially applied at the load resistance $(R_v)$ in such a manner that the measuring errors contained in the actual measuring pulse $(U_{MM})$ and caused by changes in the resistance of the supply lines (30, 36) and a temperature drift of the measuring probe (M) are eliminated.

12. A device as claimed in any one of claims 7 to 11, characterized in that the body of the measuring probe (M) is composed of two mutually insulated metallic annular elements (31, 34) which serve as a carrier of at least one emitting (32) or receiving element (37) and which, at the same time, are electrical connectors to the probe lines (36, 30).

13. A device as claimed in claim 12, characterized in that several light-emitting (32) and light-receiving elements (37) are arranged on the annular elements (31, 34), circularly around the axis of the catheter, in such a manner that the measuring angle in the cross-sectional plane of the catheter can be up to 360°.

14. A device as claimed in one of claims 7 to 13, characterized in that both electric supply lines (30, 36) to the measuring probe (M) serve at the same time as a current supply to the stimulation electrode (E).

15. A device as claimed in claim 7, characterized in that a transparent protective jacket (39), preferably of glass, surrounds the measuring probe (M).

16. A device as claimed in claim 7, characterized in that the measuring probe (M) is arranged between the electric stimulation line (30) constructed as a wire winding and the transparent insulation tube (41) in such a manner that the latter is neither interrupted nor substantially deformed.

17. A device as claimed in claim 7, characterized in that the measuring probe (M) is integrated into the stimulation catheter (K) preferably in such a manner that it can be positioned in the region of the atrioventricular valves during implantation, that is to say about 4 to 8 cm behind the stimulation electrode.

1/5

Fig. 1

Fig. 6

Fig. 7

Fig. 8

Fig. 2

Fig. 3

Fig. 4

Fig. 5

3/5

Fig. 9

Fig. 10

0 059 868

17

4/5

# Fig. 11